(19) Europäisches Patentamt
European Patent Office
Office européen des brevets

(11) **EP 0 843 546 B1**

(12) **EUROPÄISCHE PATENTSCHRIFT**

(45) Veröffentlichungstag und Bekanntmachung des Hinweises auf die Patenterteilung:
**06.10.2004   Patentblatt 2004/41**

(51) Int Cl.⁷: **A61K 7/09**

(21) Anmeldenummer: **97923883.9**

(86) Internationale Anmeldenummer:
**PCT/EP1997/002446**

(22) Anmeldetag: **13.05.1997**

(87) Internationale Veröffentlichungsnummer:
**WO 1997/047277 (18.12.1997 Gazette 1997/54)**

(54) **VERFAHREN UND MITTEL ZUR DAUERHAFTEN HAARVERFORMUNG**

PROCESS AND MEANS FOR PERMANENT HAIR-STYLING

PROCEDE, AGENTS ET INSTRUMENTS POUR REALISER DES PERMANENTES

(84) Benannte Vertragsstaaten:
**DE ES FR GB IT**

(30) Priorität: **08.06.1996  DE 19622999**

(43) Veröffentlichungstag der Anmeldung:
**27.05.1998   Patentblatt 1998/22**

(73) Patentinhaber: **Wella Aktiengesellschaft**
**64274 Darmstadt (DE)**

(72) Erfinder:
• **LANG, Günther, Dr.**
**64354 Reinheim (DE)**
• **HOCH, Dieter**
**64319 Pfungstadt/Eich (DE)**

(56) Entgegenhaltungen:
EP-A- 0 604 717      EP-A- 0 625 350
DE-A- 3 207 738      DE-A- 4 131 992

**Beschreibung**

Die vorliegende Erfindung betrifft ein Verfahren zur dauerhaften Haarverformung, dadurch gekennzeichnet, daß zunächst nur der Haaransatz fixiert und anschließend Längen und Spitzen in Form gebracht und in einer 2. Stufe fixiert werden.

Die dauerhafte Wellung eines ursprünglich glatten Haares (Dauerwelle genannt) dient bekanntlich in erster Linie dazu, einem Haarschopf mehr Fülle und Volumen zu verleihen und der Frisur eine längere Haltbarkeit zu geben.

Die klassische Technik zur Durchführung der dauerhaften Haarverformung besteht darin, daß in einer ersten Stufe die Disulfid-Bindungen des Haarkeratins mit Hilfe eines Mittels, welches einen reduzierenden Wirkstoff enthält (Verformungsmittel). geöffnet werden, sodann das Haar in die gewünschte Form gebracht wird und anschließend die Disulfid-Bindungen unter Verwendung eines einen oxidierenden Wirkstoff enthaltenden Mittels (Fixiermittel) wieder verknüpft werden. Als reduzierende Wirkstoffe werden hierbei insbesondere Sulfite, Thioglykolsäure, Thiomilchsäure, 3-Mercapto-propionsäure, Mercaptocarbonsäureester und Cystein verwendet. Als Fixiermittel werden insbesondere wasserstoffperoxidhaltige und bromathaltige Flüssigkeiten verwendet.

Üblicherweise werden die nassen Haare auf geeignete Formkörper (üblicherweise Wickler) gewickelt, dann die Wellösung aufgetragen und nach einer angemessenen Einwirkungszeit ausgespült. Dann wird die Fixierung als Flüssigkeit oder Schaum auf die gewickelten Haare gegeben, nach einer ausreichenden Einwirkungszeit ausgespült, danach wird abgewickelt und nachfixiert.

Dieses Verfahren weist jedoch eine Reihe von Nachteilen auf. Einer dieser Nachteile ergibt sich aus der unterschiedlichen Struktur der Haare vom Ansatz zur Spitze. Insbesondere Längen und Spitzen zeigen durch Alterungs-, Licht- und Feuchtigkeitseinflüsse sowie chemische Vorbehandlung wie Dauerwelle und Färbung ein anderes Umformungsverhalten. Die Folge daraus ist eine kleinlockige Umformung an den besonders strapazierten Haarspitzen und eine großlockige Umformung am ungeschädigten Haaransatz. Ein derartiges Wellbild wirkt unnatürlich und kann durch resultierende Verhakung und Vertilzung in den Spitzen zu weiteren Haarschäden führen.

Darüber hinaus sind, bedingt durch die modische Entwicklung im Frisurenstil, verbunden mit dem Zeitgeschmack, relativ starke Umformungen des Haares heute nicht mehr gefragt. Im allgemeinen Sprachgebrauch der Kunden wird in diesem Zusammenhang auch von einer zu starken und unerwünschten Krause gesprochen.

Für die Erfüllung der oben angeführten Zielsetzung einer Dauerwelle ist aber eine gute Ansatzkrause zwingend erforderlich. Strebt man diese Voraussetzung mit entsprechenden Maßnahmen wie dünne Wickler, starke Wellpräparate, längere Einwirkungszeiten etc. an, so sind in der Regel der Mittelteil des Haares und die Haarspitzen zwangsläufig zu stark gewellt - also zu kraus - und darüber hinaus wird auch die Haarstruktur über Gebühr beansprucht. Trägt man dagegen einer gewünschten großzügigen bzw. natürlichen Wellung von Mittelteil und Spitze des Haares Rechnung, ist die notwendige Ansatzkrause unzureichend, d. h. zu schwach.

Die Lehre der eigenen DE-OS 3 119 634 versucht dieses Problem dadurch zu lösen, daß in einer 2-stufigen Reduktionsbehandlung das Haar insgesamt mit einem schwachen und der Haaransatz danach mit einem verdickten stärkeren Verformungsmittel behandelt wird. Dies soll zu Haarschonung und einem gleichmäßigeren Weilbild führen. Der Nachteil dieser Vorgehensweise ist einmal eine aufwendigere Arbeitstechnik, zum anderen wird die Krausenstärke an Längen und Spitzen trotzdem verstärkt.

Es wurde nun überraschend gefunden, daß sich die genannten Nachteile durch die erfindungsgemäße Arbeitsweise vermeiden lassen und erstmals ein gleichmäßiges Verformungsbild bzw. eine wunschgemäße Gestaltung der Umformung und damit der Frisur möglich ist.

Gegenstand der vorliegenden Erfindung ist daher ein Verfahren zur dauerhaften Verformung der Haare, welches durch folgende Verfahrensschritte gekennzeichnet ist:

a) Das gewaschene und handtuchtrockene Haar wird zunächst auf Wickler, vorzugsweise übliche Dauerwellwickler, aufgewickelt.

b) Anschließend wird ein an sich bekanntes Dauerverformungsmittel auf der Basis einer haarkeratinreduzierenden Substanz - die Verformungswirksamkeit richtet sich nach der vorhandenen Haarstruktur - aufgebracht.

c) Die Einwirkungszeit wird mit einem Probewickler bestimmt und beträgt 5 bis 30 Minuten.

d) Nach Beendigung der Einwirkungszeit werden die Wickel mit Wasser abgespült und vorzugsweise die überschüssige Feuchtigkeit mit einer Saugserviette oder einem Handtuch abgetupft.

Bis zu diesem Schritt ist die Ausführung mit dem heute üblichen Wellverfahren : identisch. Die neuen und erfindungswesentlichen Schritte des vorliegenden Verfahrens sind die nachfolgenden:

e) Danach wird ein viskoses Fixiermittel auf der Basis eines Oxidationsmittels mit einer Viskosität von 50 bis 5 000 mPa·s bei 25 Grad Celsius, gemessen mit einem Haake Rotationsviskosimeter Typ VT 550 bei einem Schergeschwindigkeit von 50/Sekunde, auf die Wickel aufgetragen und es schließt sich eine Einwirkungszeit von 2 bis 6 Minuten, vorzugsweise 3 bis 5 Minuten, an.

Das viskose Fixiermittel unterscheidet sich von herkömmlichen Mitteln dieser Art durch eine so hohe Viskosität, daß am Haaransatz zwar eine gründliche Fixierung der Ansatzwellung sichergestellt ist, aber gleichzeitig ein vorzeitiges Eindringen der Fixierung in den Mittelteil und die Spitzen des aufgewickelten Haares verhindert, bzw. deutlich verzögert wird.

f) Nach dem Ablauf der Einwirkungszeit des Fixiermittels am Haaransatz wird das Haar abgewickelt und die Wickler entfernt.

Da Mittelteil und Haarspitzen zu diesem Zeitpunkt noch nicht ausreichend fixiert und stabilisiert sind, ist in diesem Zustand des Haares eine Transformation der in der Regel zu starken Wellung dieser Haarabschnitte in eine großzügige und natürliche Locke problemlos möglich.
Die "Transformation" wird wie folgt durchgeführt:

g) Die noch geschlossenen, zusammenhängenden Locken werden mit einem, vorzugsweise grobzinkigen, Kamm, den Händen oder einer Bürste aufgelockert und zur gewünschten Frisur gekämmt. Gleichzeitig wird die noch zu enge Spitzenkrause individuell und frisurenspezifisch entspannt und in eine großlockige natürliche Locke überführt. Dafür kann auch zusätzlich ein engzinkiger Kamm Verwendung finden.

In einer weiteren Ausführungsform ist es auch möglich, die abgewickelten Haare zur "Transformation" mit einem zum Stylen von Haaren bei erhöhter Temperatur geeigneten Gerät, insbesondere einer elektrisch oder durch erwärmte Luft beheizten Rundbürste, einem Lockenstab oder einem ähnlichem Formkörper oder einer Rundbürste und einem Fön zur gewünschten Frisur zu kämmen.
In einer weiteren Ausführungsform des erfindungsgemäßen Verfahrens können die Haare in der Verfahrensstufe zur "Transformation" anstelle des Kämmvorgangs auf Wickler geeigneter Dimension (z. B. Durchmesser von 15 bis 30 mm, z. B. Wasserwellwickler oder ähnliche großdimensionierte Wickler) aufgewickelt und wie nachfolgend beschrieben weiterbehandelt werden.
Da zum Zeitpunkt der Transformation die erforderliche kräftigere Ansatzwellung bereits fixiert und stabilisiert ist, bleibt diese durch die Transformation unbeeinflußt, d. h. in vollem Umfang erhalten.

h) Ist das gewünschte Wellbild erreicht, wird die restliche Haarlänge (Mittelteil und Spitze) ebenfalls mit einem Fixiermittel auf der Basis eines Oxidationsmittels behandelt.

Zu diesem Zweck kann entweder das viskose Fixiermittel in das gesamte Haar eingearbeitet (z. B. eingekämmt) werden oder auch eine handelsübliche dünnflüssige Fixierlösung (Spülfixierung) zum Einsatz kommen. In letzterem Fall handelt es sich um eine 2-Komponenten-Fixierung, mit dem Vorteil einer Zeitersparnis.

i) Nach einer weiteren Einwirkungszeit von 1 bis 10 Minuten, vorzugsweise 3 bis 5 Minuten, wird das Fixiermittel mit Wasser ausgespült.

Bei der Verwendung einer zusätzlichen dünnflüssige Fixierlösung für die Längen der Haare kann eine Einwirkungszeit entfallen.
Als Haarverformungsmittel sind alle üblichen derartigen Mittel geeignet. Das Verformungsmittel kann sowohl in Form einer wäßrigen Lösung oder einer Emulsion als auch in verdickter Form auf wäßriger Basis, insbesondere als Creme, Gel oder Paste vorliegen.
Die in dem Dauerverformungsmittel enthaltenen haarkeratinreduzierenden Substanzen werden in dem gebrauchsfertigen Mittel zur dauerhaften Haarverformung bevorzugt in einer Menge von 2 bis 20 Gewichtsprozent, besonders bevorzugt in einer Menge von 4 bis 12 Gewichtsprozent und ganz besonders bevorzugt in einer Menge von 6 bis 10 Gewichtsprozent eingesetzt.
Als haarkeratinreduzierende Substanzen können z. B. Thioglykolsäure, Thiomilchsäure, Cystein, Cysteamin, Alkyl- oder Acylcysteamine oder die Salze dieser Verbindungen, ferner Thioglykolsäureester oder Sulfite, insbesondere im sauren pH-Bereich, auch im Gemisch miteinander, eingesetzt werden.
Die gebrauchsfertigen Dauerverformungsmittel besitzen bevorzugt einen pH-Wert von 6 bis 9 besonders bevorzugt von 6,5 bis 8,5. Als Alkalisierungsmittel bzw. als Mittel zur Einstellung des pH-Wertes kommen insbesondere Ammoniak oder Natronlauge, aber auch alle anderen wasserlöslichen, physiologisch verträglichen Salze von organischen und anorganischen Basen, wie z.B. Ammoniumhydrogencarbonat, in Betracht.

Das Dauerverformungsmittel kann als Ein-, Zwei- oder Dreikomponenten-Präparat konfektioniert sein, wobei das Mittel sowohl in Form einer wäßrigen Lösung oder einer Emulsion als auch in verdickter Form auf wäßriger Basis, insbesondere als Creme, Gel oder Paste, vorliegen kann.

So ist das Dauerverformungsmittel zum Beispiel durch Vermischen zweier Komponenten erhältlich, von denen die erste Komponente mindestens ein Alkalisierungsmittel, beispielsweise ein Alkalicarbonat, Ammoniumcarbonat, Alkalihydrogencarbonat oder Ammoniumhydrogencarbonat. und die haarkeratinreduzierende Substanz enthält und die zweite Komponente mindestens eine der nachstehend genannten kosmetischen Zusatzstoffe und Wasser enthält.

Ebenfalls ist es möglich, das Dauerverformungsmittel in Form eines Dreikomponenten-Präparates abzupakken, wobei eine Komponente einige der nachfolgend genannten kosmetischen Zusatzstoffe sowie Wasser enthält, eine zweite, wasserfreie Komponente die haarkeratinreduzierende Substanz enthält und die dritte Komponente weitere Zusatzstoffe, wie z. B. Parfümöle, Lösungsvermittler und Pflegestoffe, in wäßriger Lösung oder in wasserfreier Form enthält.

Bei allen Ausführungsformen des Dauerverformungsmittels können die kosmetischen Zusatzstoffe sowohl in der wäßrigen als auch in der/den nichtwäßrigen Komponente(n) enthalten sein.

Selbstverständlich kann das Dauerverformungsmittel alle für derartige Mittel üblichen und bekannten Zusatzstoffe, zum Beispiel Verdickungsmittel, wie beispielsweise, Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quatemäre Ammoniumsalze, Alkylbetaine, oxethylierte Alkylphenole, Fettsäurealkanolamide oder oxethylierte Fettsäureester; femer Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen. Parfümöle, Farbstoffe sowie haarkonditionierende und haarpflegende Bestandteile, wie zum Beispiel kationische Polymere, Lanotinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0.01 bis 1 Gewichtsprozent, die Puffersubstanzen in einer Menge von insgesamt 0.1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder lmidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsäure, die Dithiole der genannten Verbindungen oder die jeweiligen Salze, ebenfalls in einer Menge von 1 bis 30 Gewichtsprozent, zugesetzt werden.

Nach einer für die dauerhafte Verformung des Haares ausreichenden Einwirkungszeit, welche je nach Haarbeschaffenheit, dem pH-Wert und der Verformungswirksamkeit des Verförmungsmittefs sowie in Abhängigkeit von der Anwendungstemperatur 5 bis 30 Minuten (20 bis 30 Minuten ohne Wärmeeinwirkung; 15 bis 25 Minuten mit Wärmeeinwirkung) beträgt, wird das Haar mit Wasser gespült. Die Einwirkungszeit des Dauerverformungsmittels bei normalem, bisher oxidativ unbehandeltem Haar beträgt 20 bis 25 Minuten und bei oxidativ behandeltem Haar 15 bis 20 Minuten. Vorzugsweise dauert der Ausspülvorgang 2 bis 4 Minuten. Danach ist es vorteilhaft, die Wickel mit einer Saugserviette oder einem Handtuch abzutupfen. Die Anwendungstemperatur bei Wärmeeinwirkung liegt im Bereich von 30 bis 45 Grad Celsius.

Das viskose Fixiermittel ist dadurch gekennzeichnet, daß es eine solche Viskosität aufweist, daß ein vorzeitiges Eindringen der Fixierung in den Mittelteil und die Spitzen des aufgewickelten Haares verhindert bzw. deutlich verzögert wird, d.h. es liegt in verdickter Form auf wäßriger Basis, insbesondere als Creme, Gel, Suspension, Paste oder als viskoser Schaum vor. Vorzugsweise weist es eine Viskosität von 50 bis 5 000 mPa·s bei 25 Grad Celsius, besonders bevorzugt 100 bis 3 000 mPa·s bei 25 Grad Celsius und ganz besonders bevorzugt 100 bis 1000 mPa·s bei 25 Grad Celsius, gemessen z. B. mit einem Haake Rotationsviskosimeter Typ VT 550 bei einer Schergeschwindigkeit von 50/Sekunde, auf.

Als Verdicker kommen sowohl polymere Verdickungsmittel wie Polyacrylsäure und deren Derivate, Cellulosederivate, Alginate und Chitin- oder Chitosanderivate als auch tensidische Systeme (Emulsionen, Mikroemulsionen, Suspensionen und viskose flüssigkristalline Phasen) wie Kombinationen geeigneter Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quatemäre Ammoniumsalze, Alkylbetaine, oxethylierte Alkylphenole, oxethylierte Fettalkohole, Fettsäurealkanolamide oder oxethylierte Fettsäureester mit geeigneten Fettphasen wie Fettalkoholen, Fettsäureamiden, Fettsäureglyceriden und Fettsäureglycolaten, Vaseline und Paraffinölen und -wachsen, als auch anorganische Verdickungsmittel wie Bentoniten in Frage. Die Verdicker sind vorzugsweise in einer Menge von insgesamt 1 bis 30 Gew.% enthalten.

Beispiele für in Fixiermitteln verwendbare Oxidationsmittel sind Kalium- und Natriumbromat, Natriumperborat, Hamstoffperoxid und Hydrogenperoxid. Die Konzentration des Oxidationsmittels ist in Abhängigkeit von der Art des Oxidationsmittels, der Anwendungszeit (in der Regel 5 bis 15 Minuten) und der Anwendungstemperatur unterschiedlich. Normalerweise liegt das Oxidationsmittel in dem gebrauchsfertigen viskosen Fixiermittel in einer Konzentration von 1 bis 15 Gewichtsprozent vor. Bevorzugt enthält das viskose Fixiermittel Wasserstoffperoxid in einer Menge von 1 bis 5 Gew. % oder Bromat in einer Menge von 5 bis 15 Gew. %.

Das Mittel für die oxidative Nachbehandlung kann selbstverständlich weitere Stoffe, wie zum Beispiel Netzmittel, Pflegestoffe wie kationische Polymere, schwache Säuren, Puffersubstanzen oder Peroxidstabilisatoren, enthalten.

Zur Durchführung der Verfahrensstufe h) kann entweder das viskose Fixiermittel in das gesamte Haar eingearbeitet werden, oder aber jedes beliebige, bisher für eine derartige Behandlung verwendete, Fixiermittelmittel angewandt werden.

Die nachfolgenden Beispiele sollen den Gegenstand der Erfindung näher erläutern, ohne den Gegenstand auf diese Beispiele zu beschränken.

**BEISPIELE**

**Beispiel 1**

Ungeschädigtes Haar wird mit einem Shampoo gewaschen, frottiert und auf Wickler mit einem Durchmesser von 8 Millimetern gewickelt. Anschließend wird die nachstehend angeführte Dauerwellösung vom pH = 8,2 mittels einer Flasche mit Auftragedüse gleichmäßig auf dem gewickelten Haar verteilt.

| Dauerwellösung | |
|---|---|
| 10,00 g | Thioglykolsäure |
| 6,10 g | Ammoniumhydrogencarbonat |
| 7,60 g | Ammoniak, 25%ige wäßrige Lösung |
| 1,00 g | mit 35 Mol Ethylenoxid oxethyliertes Rizinusöl |
| 0,20 g | Cocoamphoglycinat (CTFA: SODIUM COCOAMPHOACETATE) |
| 0,50 g | Parfümöl |
| 74,60 g | Wasser |
| 100,0 g | |

Sodann wird das Haar mit einer Plastikhaube abgedeckt und 10 Minuten lang unter einer Trockenhaube bei einer Temperatur von 40 Grad Celsius erwärmt. Anschließend wird die Abdeckung entfernt, das aufgewickelte Haar 2 bis 3 Minuten lang mit warmem Wasser gespült und die überschüssige Feuchtigkeit mittels eines Frotteehandtuchs von den Wickeln abgetupft. Danach werden 60 g des folgenden viskosen Fixiermittels auf die Wickel mit einem Pinsel aufgetragen:

| viskoses Fixiermittel mit Wasserstoffperoxid | |
|---|---|
| 2,00 g | Cetylstearylalkohol |
| 0,20 g | Dinatriumphosphat |
| 0.15 g | o-Phosphorsäure |
| 0,20 g | Natriumlaurylsulfat |
| 4,00 g | Wasserstoffperoxid, 50 Gew. %-ig |
| 0,30 g | Parfümöl |
| 0,10 g | Vinylpyrrolidon/Styrol-Mischpolymerisat (Antara® 430 der GAF Corp.; New York/USA) |
| 93,05 g | Wasser |
| 100,00 g | |

Es folgt eine Einwirkungszeit von 3 Minuten.

Nach dem Ablauf der Einwirkzeit werden die Wickler abgewickelt und die mit Ausnahme des Haaransatzes noch nicht fixierte, relativ starke Wellung in den Längen der Haare mit einem Kamm in die gewünschte Stärke der Umformung transformiert. Ist der gewünschte Zustand erreicht, werden weitere 50 g der vorstehenden viskosen Fixiermittels in das gesamte Haar verteilt und eingearbeitet. Es folgt eine weitere Einwirkungszeit von 5 Minuten. Danach wird das Haar erneut mit warmem Wasser gründlich ausgespült. Damit ist die eigentliche Behandlung beendet. Es schließt sich die gewohnte Erstellung der Frisur mit Fön und Bürste an.

**Beispiel 2**

Oxidativ geschädigtes und dauergewelltes Haar wird mit einem Shampoo gewaschen, frottiert und auf Wickler mit einem Durchmesser von 8 Millimetern gewickelt.
Anschließend wird die nachfolgende Dauerwellösung gleichmäßig auf dem gewickelten Haar verteilt.

**neutrale Dauerwellösung aus 2 Komponenten**

| Komponente A | |
|---|---|
| 94,6 g | Ammoniumthioglykolat, 70% ige wäßrige Lösung |
| 5,4 g | Cysteinhydrochlorid |
| 100,0 g | |
| (pH = 6,0) | |

| Komponente B | |
|---|---|
| 0,8 g | Ammoniak, 25%ige wäßrige Lösung |
| 0,5 g | Ammoniumhydrogencarbonat |
| 2,0 g | Laurylalkohol, mit 4 Mol Ethylenoxid oxethyliert |
| 1,0 g | Polydimethyldiallylammoniumchlorid |
| 1,0 g | Parfümöl |
| 0,5 g | Vinylpyrrolidon-Styrol-Mischpolymerisat |
| 0,5 g | Cetyltrimethylammoniumchlorid |
| 93.7 g | Wasser |
| 100,00 g | |

Die Komponente B weist einen pH-Wert von 8,5 auf. Vor dem Gebrauch werden 15 g der Komponente A und 66 g der Komponente B zu 81 g des gebrauchsfertigen Haarverformungsmittels vom pH = 7,5 vermischt.
Sodann wird das Haar mit einem Infrarot-Wärmehaube mit der Temperatureinstellung "Dauerwelle" 10 Minuten lang behandelt. Anschließend wird das aufgewickelte Haar 2 bis 3 Minuten lang mit warmem Wasser gespült und die überschüssige Feuchtigkeit mittels eines Frotteehandtuchs von den Wickeln abgetupft. Danach werden 60 g des nachfolgenden viskosen Fixiermittels auf die Wickel mit einem Pinsel aufgetragen.

| viskoses Fixiermittel mit Bromat | |
|---|---|
| 11,0 g | Natriumbromat |
| 6,0 g | Cetylstearylalkohol |
| 2.0 g | Vaseline |
| 2,0 g | mit 25 Mol Ethylenoxid oxethylierter Cetylstearylalkohol (CTFA: Ceteareth 25) |
| 1,0 g | Cetyltrimethylammoniumchlorid |
| 1,0 g | Poly(dimethyldiallylammoniumchlorid) |
| 0,3 g | Parfümöl |
| 0,5 g | Natriumphosphat |
| 1,0 g | Dinatriumphosphat |
| 0,1 g | Vinylpyrrolidon/Styrol-Mischpolymerisat (Antara® 430 der GAF Corp.; New York/USA) |

(fortgesetzt)

| viskoses Fixiermittel mit Bromat | |
|---|---|
| 75,1 g | Wasser |
| 100,0 g | |

Es folgt eine Einwirkungszeit von 3 Minuten. Nach dem Ablauf der Einwirkungszeit werden die Wickler abgewickelt und die mit Ausnahme des Haaransatzes noch nicht fixierte, relativ starke Wellung in den Längen der Haare mit einem Kamm in die gewünschte Stärke der Umformung transformiert. Ist der gewünschte Zustand erreicht, werden weitere 50 g des vorstehenden viskosen Fixiermittels in das gesamte Haar verteilt und eingearbeitet. Es folgt eine weitere Einwirkungszeit von 5 Minuten. Danach wird das Haar erneut mit warmem Wasser gründlich ausgespült. Damit ist die eigentliche Behandlung beendet. Das so geformte Haar wird ohne zusätzliche mechanische Hilfsmittel unter der Infrarot-Wärmehaube luftgetrocknet.

**Patentansprüche**

1. Verfahren zur dauerhaften Verformung von Haaren, **dadurch gekennzeichnet, daß** man

   a) das Haar auf Wickler aufwickelt,

   b) auf das gewickelte Haar ein Dauerverformungsmittel auf der Basis einer haarkeratinreduzierenden Substanz aufbringt,

   c) das Dauerverformungsmittel 5 bis 30 Minuten lang auf das Haar einwirken läßt,

   d) die Wickel mit Wasser abspült,

   e) ein viskoses Fixiermittel auf der Basis eines Oxidationsmittels mit einer Viskosität von 50 bis 5 000 mPa·s bei 25 Grad Celsius, gemessen mit einem Haake Rotationsviskosimeter Typ VT 550 bei einem Schergeschwindigkeit von 50/Sekunde, auf die Wickel aufträgt und 2 bis 6 Minuten lang einwirken läßt,

   f) das Haar abwickelt und die Wickler entfernt.

   g) zur Transformation der zu starken Umformung in eine großlockige Welle das Haar entweder

      g1) mit einem Kamm, den Händen oder einer Bürste in die gewünschte Frisur kämmt und gleichzeitig die noch zu enge Spitzenkrause entspannt und in eine großlockige natürliche Locke überführt oder

      g2) mit einem zum Stylen von Haaren bei erhöhter Temperatur geeigneten Gerät, insbesondere einer elektrisch oder durch erwärmte Luft beheizten Rundbürste, einem Lockenstab oder einem ähnlichem Formkörper oder einer Rundbürste und einem Fön zur gewünschten Frisur kämmt oder

      g3) auf Wickler mit einem Durchmesser von 15 bis 30 mm wickelt,

   h) die restliche Haarlänge (Mittelteil und Spitze) mit einem Fixiermittel auf der Basis eines Oxidationsmittels behandelt,

   i) nach einer Einwirkungszeit von 1 bis 10 Minuten das Fixiermittel mit Wasser aus dem Haar ausspült.

2. Verfahren nach Anspruch 1, **dadurch gekennzeichnet, daß** der Verfahrensschritt c) bei erhöhter Temperatur, vorzugsweise bei 30 bis 45 Grad Celsius, ausgeführt wird.

3. Verfahren nach Anspruch 1 oder 2, **dadurch gekennzeichnet, daß** man zur Erzielung der erhöhten Temperatur ein Infrarot-Strahlungsgerät verwendet.

4. Verfahren nach einem der Ansprüche 1 bis 3, **dadurch gekennzeichnet, daß** die Einwirkungszeit des Dauerverformungsmittels bei normalem, bisher oxidativ unbehandeltem Haar 20 bis 25 Minuten und bei oxidativ behandeltem Haar 15 bis 20 Minuten beträgt.

5. Verfahren nach einem der Ansprüche 1 bis 4, **dadurch gekennzeichnet, daß** die genaue Einwirkungszeit des Dauerverformungsmittels in an sich bekannter Weise mit einem Probewickler bestimmt wird.

6. Verfahren nach einem der Ansprüche 1 bis 5, **dadurch gekennzeichnet, daß** das Haar nach der Verfahrensstufe d) mit einer Saugserviette oder einem Handtuch abgetupft wird.

7. Verfahren nach einem der Ansprüche 1 bis 6, **dadurch gekennzeichnet daß** zur Durchführung der Verfahrensstufe h) das viskose Fixiermittel verwendet wird.

8. Verfahren nach einem der Ansprüche 1 bis 7, **dadurch gekennzeichnet, daß** man das viskose Fixiermittel 3 bis 5 Minuten einwirken läßt.

**Claims**

1. Process for permanent hairstyling, **characterized in that**

   a) the hair is wound onto rollers,

   b) a permanent styling means based on a hair keratin-reducing substance is applied to the wound hair,

   c) the permanent styling agent is left to act on the hair for 5 to 30 minutes,

   d) the rollers are rinsed with water,

   e) a viscous neutralizer based on an oxidizing agent with a viscosity of from 50 to 5000 mPa.s at 25 degrees Celsius, measured using a Haake rotary viscometer model VT 550 at a shear velocity of 50/second, is applied to the rollers and left to act for 2 to 6 minutes,

   f) the hair is unwound and the rollers are removed,

   g) to transform the excessively strong shaping to a large-curled wave the hair is either

      g1) combed with a comb, the hands or a brush into the desired style and at the same time the curl on the ends, which is still too tight, is relaxed and converted to a large-curled natural lock or

      g2) combed with a device suitable for styling hair at elevated temperature, in particular a round brush heated electrically or by warmed air, curling tongs or a similar shaped article or a round brush and a hairdryer to give the desired hairstyle or

      g3) wound onto rollers with a diameter of from 15 to 30 mm,

   h) the remaining hair length (middle section and ends) are treated with a neutralizer based on an oxidizing agent,

   i) after a contact time of from 1 to 10 minutes, the neutralizer is rinsed out of the hair with water.

2. Process according to Claim 1, **characterized in that** process step c) is carried out at elevated temperature, preferably at 30 to 45 degrees Celsius.

3. Process according to Claim 1 or 2, **characterized in that** an infrared radiation device is used to achieve the elevated temperature.

4. Process according to one of Claims 1 to 3, **characterized in that** the contact time of the permanent styling means is 20 to 25 minutes for normal, hitherto oxidatively untreated hair, and is 15 to 20 minutes for oxidatively treated hair.

**5.** Process according to one of Claims 1 to 4, **characterized in that** the precise contact time of the permanent styling means is determined in a manner known per se using a sample roller.

**6.** Process according to one of Claims 1 to 5, **characterized in that** the hair is dabbed after process step d) with an absorbent napkin or a hand towel.

**7.** Process according to one of Claims 1 to 6, **characterized in that** the viscous neutralizer is used to carry out process step h).

**8.** Process according to one of Claims 1 to 7, **characterized in that** the viscous neutralizer is left to act for 3 to 5 minutes.

**Revendications**

**1.** Procédé pour la mise en forme permanente des cheveux, **caractérisé en ce que**

a) on enroule les cheveux sur des rouleaux,
b) on applique sur les cheveux enroulés une composition de mise en forme permanente à base d'une substance réduisant la kératine des cheveux,
c) on laisse agir sur les cheveux la composition de mise en forme permanente pendant 5 à 30 minutes,
d) on rince les rouleaux à l'eau,
e) on applique sur les rouleaux un fixateur visqueux à base d'un oxydant, ayant une viscosité de 50 à 5 000 mPa.s à 25°C, mesurée à l'aide d'un viscosimètre rotatif Haake type VT 550, à une vitesse de cisaillement de 50/seconde, et on laisse agir pendant 2 à 6 minutes,
f) on déroule les cheveux et on retire les rouleaux,
g) pour la transformation de la forte mise en forme en une ondulation à grandes boucles, les cheveux sont soit

g1) coiffés en la coiffure désirée, au moyen d'un peigne, des mains ou d'une brosse, et en même temps la frisure des pointes encore trop serrée est relâchée et transformée en une grande boucle naturelle ou
g2) coiffés en la coiffure désirée, au moyen d'un appareil approprié au coiffage des cheveux à température élevée, en particulier d'une brosse ronde chauffée électriquement ou à l'air chauffé, d'un fer à friser ou d'un dispositif de mise en forme similaire ou d'une brosse ronde et d'un sèche-cheveux, ou
g3) enroulés sur des rouleaux ayant un diamètre de 15 à 30 mm,

h) la longueur restante des cheveux (partie moyenne et pointe) est traitée par un fixateur à base d'un oxydant,
i) après un temps d'action de 1 à 10 minutes, le fixateur est éliminé des cheveux par rinçage à l'eau.

**2.** Procédé selon la revendication 1, **caractérisé en ce que** l'étape de processus c) est effectuée à température élevée, de préférence à une température de 30 à 45°C.

**3.** Procédé selon la revendication 1 ou 2, **caractérisé en ce que**, pour l'obtention de la température élevée, on utilise un appareil à rayonnement infrarouge.

**4.** Procédé selon l'une quelconque des revendications 1 à 3, **caractérisé en ce que** le temps d'action de la composition de mise en forme permanente va de 20 à 25 minutes dans le cas de cheveux normaux, jusque-là non traités par oxydation, et de 15 à 20 minutes dans le cas de cheveux traités par oxydation.

**5.** Procédé selon l'une quelconque des revendications 1 à 4, **caractérisé en ce que** le temps d'action exact de la composition de mise en forme permanente est déterminé de façon connue à l'aide d'un rouleau d'essai.

**6.** Procédé selon l'une quelconque des revendications 1 à 5, **caractérisé en ce que** les cheveux sont essuyés avec une serviette absorbante ou une serviette de toilette, après l'étape de processus d).

**7.** Procédé selon l'une quelconque des revendications 1 à 6, **caractérisé en ce que** pour effectuer l'étape de processus h) on utilise le fixateur visqueux.

**8.** Procédé selon l'une quelconque des revendications 1 à 7, **caractérisé en ce qu'**on laisse agir le fixateur visqueux pendant 3 à 5 minutes.